# EUROPEAN PATENT APPLICATION

(11) **EP 2 191 851 A1**
(43) Date of publication of application: **02.06.2010**
(21) Application number: 08832377.9
(22) Date of filing: 05.09.2008
(51) Int. Cl.: A61L 27/00

(54) **PROCESS FOR PRODUCING MATERIAL FOR HARD TISSUE CONTACT TOOL, AND HARD TISSUE CONTACT TOOL**

(30) Priority: 18.09.2007 JP 2007241100
(71) Applicant: National University Corporation Tokyo Medical and Dental University, Tokyo 113-8510 (JP)
(72) Inventor: TSUTSUMI, Yusuke, Tokyo 113-8510 (JP); HANAWA, Takao, Tokyo 113-8510 (JP); NISHIMURA, Daichi, Tokyo 113-8510 (JP)
(74) Representative: Ström & Gulliksson AB
(86) International application number: PCT/JP2008/066054
(87) International publication number: WO 2009/037983

(57) **Abstract**

This invention provides a process for producing a material for a hard tissue contact tool, which can satisfactorily impart functionality and can improve surface treatment efficiency, and a hard tissue contact tool. The process for producing a material for a hard tissue contact tool comprises the step of subjecting an electroconductive material having a surface formed of at least one material selected from the group consisting of zirconium, a zirconium compound, and a zirconium alloy to cathode polarization treatment in an aqueous electrolyte solution containing an effective amount of hydrogen peroxide. The production process can produce a material for a hard tissue contact tool which can realize efficient precipitation of calcium phosphate on its surface upon implantation in a living body.

## Description

### TECHNICAL FIELD

The present invention relates to a process for producing a material for a hard tissue contact device implanted in a living body, and a hard tissue contact device.

### BACKGROUND ART

Devices such as dental implants and hip prosthesis stems to be in direct contact with a hard tissue are required to be capable of rapid fusion with the hard tissue. In addition, it is necessary for scaffolding devices used in tissue engineering to have characteristics which permits quick bone formation, on its surface.

In order to meet such demands, a surface treatment for imparting functionality to device materials has been carried out. A surface treatment method which has been conventionally carried out predominantly is an alkali treatment method which includes the steps of immersing the device material in a phosphate solution, and thereafter immersing the same in a phosphoric acid solution (for example, see Patent Document 1).

According to this method, by immersing a device material in a phosphate solution, a large amount of calcium phosphate is precipitated on the surface of the device material when brought into contact with a phosphoric acid solution. Thus, the device material can rapidly fuse with a hard tissue, and/or the bone can be quickly formed.

Patent Document 1: Japanese Translation of PCT International Publication, Publication No. 2005-503850

### DISCLOSURE OF THE INVENTION

### Problems to be Solved by the Invention

However, in order to achieve the effects as described above according to the alkali treatment method, it is necessary to carry out the immersion in an alkaline solution for a time period as long as one or more days, and heating after the immersion for several days. Thus, treatment efficiency has been inferior when satisfactorily imparting functionality is intended because of step prolongation.

The present invention was made in view of the foregoing circumstances, and an object of the invention is to provide a process for producing a material for a hard tissue contact device, which can satisfactorily impart functionality and can improve surface treatment efficiency, and the hard tissue contact device.

### Means for Solving the Problems

The present inventors found that a cathode polarization treatment carried out in an electrolyte solution containing hydrogen peroxide enables the treatment efficiency to be improved, and the treatment time to be shortened. Accordingly, the present invention was completed. Specifically, the present invention provides the following.

A first aspect of the present invention provides a process for production of a material for a hard tissue contact device, the process including: subjecting an electroconductive material having a surface formed of at least one selected from the group consisting of zirconium, a zirconium compound, and a zirconium alloy to a cathode polarization treatment in an aqueous electrolyte solution containing an effective amount of hydrogen peroxide.

According to the first aspect of the invention, since the following reactions are caused by carrying out a cathode polarization treatment, the electroconductive material is alkalized in the vicinity of the surface thereof:
O₂+2H₂O+4_{e⁻→}4OH⁻; and
2H₂O+2e⁻→H₂→2OH⁻

However, hydrogen gas is generated as a byproduct, and this hydrogen gas may be absorbed into the electroconductive material, resulting in possible risks of: embrittlement of the electric conductivity material; inhibiting polarization; and disturbing the alkaline condition in the vicinity of the surface of the electroconductive material due to a convection phenomenon.

In this respect, according to the first aspect of the invention, since an effective amount of hydrogen peroxide is contained in the aqueous electrolyte solution, hydrogen gas is consumed in accordance with the following reaction: H₂+H²O₂→2H₂O

In addition, the following reactions promote alkalization:

H₂O₂+2e⁻→2OH₋

; and

HO_{2⁻}+H₂O+2e⁻→3OH₋

Thus, the material for a hard tissue contact device can satisfactorily impart functionality when implanted in a living body, since a large amount of calcium phosphate precipitates on its surface. In addition, the cathode polarization treatment allows the aforementioned functionality to be achieved in a significantly shorter period of time as compared with conventional alkali treatment methods; therefore, the surface treatment efficiency can be greatly improved.

A second aspect of the invention provides the process according to the first aspect in which the electrolyte solution is an aqueous solution containing sulfate.

When a chloride ion, a fluoride ion, a carbonate ion and the like are contained in an electrolyte solution, there is a risk of corrosion of a zirconium based surface upon a cathode polarization treatment.

In this respect, according to the second aspect of the invention, since sulfate is employed as the electrolyte, corrosion of the electroconductive material upon a cathode polarization treatment is suppressed. Thus, a stronger material for a hard tissue contact device can be produced.

A third aspect of the present invention provides a hard tissue contact device formed with the material produced by the process according to the first or second aspect.

According to the third aspect of the invention, similar effects to those of the first or second aspect are achieved.

A forth aspect of the present invention provides a method for promoting precipitation of calcium phosphate on a surface constituted with at least one selected from the group consisting of zirconium, a zirconium compound, and a zirconium alloy in a living body environment, the method including:
subjecting the surface to a cathode polarization treatment in an aqueous electrolyte solution containing an effective amount of hydrogen peroxide.

### Effects of the Invention

According to the present invention, an electroconductive material is alkalized in the vicinity of the surface by subjecting to a cathode polarization treatment. Upon the treatment, since an effective amount of hydrogen peroxide is contained in the aqueous electrolyte solution, a byproduct, hydrogen gas, is consumed. Therefore, when implanted in a living body, the material can satisfactorily impart biological functionality, i.e., precipitation of a large amount of calcium phosphate on the surface. In addition, the cathode polarization treatment allows the aforementioned functionality to be achieved in a significantly shorter period of time as compared with conventional alkali treatment methods; therefore, the surface treatment efficiency can be greatly improved.

### BRIEF DESCRIPTION OF THE DRAWINGS

Fig. 1 shows micrographs illustrating surface states of the materials for a hard tissue contact device according to Examples of the present invention and Comparative Examples; and
Fig. 2 shows a micrograph illustrating a surface state of the material for a hard tissue contact device according to Comparative Example.

### PREFERRED MODE FOR CARRYING OUT THE INVENTION

Hereinafter, embodiments of the present invention are explained, but limitation of the present invention thereto is not intended.

The process for production of a material for a hard tissue contact device of the present invention includes the step of subjecting an electroconductive material to a cathode polarization treatment in an aqueous electrolyte solution.

The electroconductive material used in the present invention has a surface formed of at least one selected from the group consisting of zirconium, a zirconium compound, and a zirconium alloy. The "zirconium compound" herein means a compound containing zirconium as an element, and for example, zirconium oxide may be included. Furthermore, the "zirconium alloy" refers to an alloy of zirconium or the zirconium compound with other elemental metal or a compound containing other element, and for example, alloys of zirconium typified by a zircaloy alloy, which is formed by addition of tin, iron, chromium, niobium or the like, and zirconium bulk metal glasses including zirconium as a principal component (alloys of zirconium formed by addition of copper, nickel, aluminum, niobium or the like) may be included. Examples of the other element include elements that are highly safe in living bodies such as titanium and tantalum, and elements to be added in a slight amount for improving corrosion resistance such as platinum and palladium.

Since such zirconium based electroconductive materials generally have greater strength and superior stretchability than materials such as titanium, a highly versatile material for a hard tissue contact device can be produced. The "surface" referred to herein may be either the surface of the electroconductive material itself, or a coating formed on the material.

The electrolyte solution used in the present invention contains an effective amount of hydrogen peroxide. The term "effective amount" herein refers to the amount of hydrogen peroxide required for decreasing the amount of the generated hydrogen gas to a desired level, and minimum amounts which can be present as an impurity by chance should be excluded. The effective amount may be determined appropriately depending on the conditions, but excess amounts may result in absorption of hydrogen peroxide itself into the electroconductive material to make the electric conductivity material brittle, whereas insufficient amounts lead to failure in satisfactory decrease of hydrogen gas. Taking into consideration these, the effective amount is preferably no less than 0.01 mass% and no greater than 1 mass%, in general.

Addition of hydrogen peroxide to the electrolyte is generally conducted prior to the cathode polarization treatment; however, the addition may be conducted continuously or intermittently during cathode polarization.

The electrolyte solution contains a supporting electrolyte in addition to hydrogen peroxide. As such a supporting electrolyte, commonly used neutral supporting electrolytes such as sodium sulfate and sodium chloride may be exemplified, and sulfate (e.g., sodium sulfate, potassium sulfate and magnesium sulfate) are preferred in light of pertinence to the treatment of zirconium based surfaces.

The concentration of the supporting electrolyte is preferably no less than 0.1 mol/L taking into consideration that deficient supporting electrolyte may lead to insufficient electric conductivity of the electrolyte solution. In addition, when a chloride ion, a fluoride ion, a carbonate ion or the like is included in the electrolyte solution, the concentration should be appropriately set since an excessively high concentration may result in corrosion of the electrode.

Taking into account that excessively high voltage applied in the cathode polarization treatment leads to generation of hydrogen gas exceeding the allowable value, whereas deficient voltage leads to need of a significantly long time for the surface treatment, the voltage applied is preferably no lower than -1 V and no higher than -5 V relative to the standard hydrogen electrode. Moreover, taking into account that excessively high electric current density with respect to the surface area of the cathode leads to generation of hydrogen gas exceeding the allowable value, whereas deficient density leads to insufficient electric conductivity of the electrolyte solution, the electric current density with respect to the surface area of the cathode is preferably no lower than 1 x 10^{- 2} A/dm² and no higher than 1 x 10¹ A/dm².

Since the reactions as in the following occur when the cathode polarization treatment as described above is carried out, the electroconductive material is alkalized in the vicinity of the surface.

O₂2H₂O+4e⁻→4OH₋

; and

2H₂+2e⁻→H₂→2OH⁻

In addition, hydrogen gas which may be generated as a byproduct is consumed by hydrogen peroxide according to the following reaction.

H₂+H₂O₂→2H₂O

Moreover, the alkalization is promoted by the following reactions.

H₂O₂+2e⁻→2OH₋

; and

HO_{2⁻}+H₂O+2e₋→3Oh⁻

Accordingly, events of: making the electric conductivity material brittle; inhibiting polarization; and disturbing the alkaline condition in the vicinity of the surface of the electroconductive material due to a convection phenomenon can be avoided, which may occur through absorption of the hydrogen gas into the electroconductive material.

Since such a cathode polarization treatment is carried out in a state in which the electric conductivity material is immersed in the electrolyte solution, a material for a hard tissue contact device having the entire surface treated efficiently and uniformly can be produced in a short period of time. Although it is necessary to immerse a material in an alkaline solution for usually about one day according to alkali treatment methods in fact, the cathode polarization procedure is completed generally within about one hour. Furthermore, according to ion implantation methods by way of ion beams, and plasma spraying methods with hydroxyapatite, an insufficiently treated surface portion is likely to occur when the material has a complicated shape. However, the cathode polarization treatment enables a uniform surface treatment even though the electroconductive material has a complicated shape, due to alkalization of the vicinity of the entire surface.

It should be noted that the preset temperature during the cathode polarization treatment is not particularly limited, and room temperature is generally acceptable. However, the preset temperature is preferably about 5°C when suppression of absorption of hydrogen into the electric conductivity material is particularly needed.

By subjecting thus produced material for a hard tissue contact device to processing so as to have appropriate shape and dimensions, a hard tissue contact device can be manufactured. Since this hard tissue contact device allows calcium phosphate to precipitate on the surface in living body environments, it can rapidly fuse with a hard tissue, or can quickly permit bone formation.

The hard tissue contact device of the present invention may be used under conditions to be in contact with a hard tissue after implantation in a living body, and can be preferably used as, for example, dental implants, artificial dentures, bridges, crown restoration matters (fillers, restoration materials, etc.), plates, bolts, screws, and the like. In the case of plasma spraying methods and the like in which the surface is coated with hydroxyapatite or the like, when applied to bone plates and the like used following deformation of the shape prior to implantation thereof, as well as to dental implants, screws and the like to which an enormous force is applied on the surface upon implantation, the coated layer may separate before implantation, whereby satisfactory performance may not be achieved after the implantation. However, according to the hard tissue contact device of the present invention, since calcium phosphate spontaneously precipitates on the surface after the implantation, biological functionality can be sufficiently exerted after implantation in a living body.

### EXAMPLES

### Example

First, a cylinder (diameter: 8 mm) made of zirconium having a purity of 99.6% was cut at 2 mm intervals along an axial direction, and the cut surfaces were polished using abrasive paper with a ground particle grade of #320 to #800. Thereafter, polishing with a diamond paste having a particle size of 9 µm, a subsequent diamond paste having a particle size of 3 µm, and a 0.04 µm silica paste for final finishing was carried out, whereby mirror polishing of the surface of the electroconductive material was completed.

Next, using the electroconductive material as a working electrode, a platinum electrode as a counter electrode, and a saturated calomel electrode as a reference electrode, these electrodes were immersed in an electrolyte solution (supporting electrolyte: 0.5 mol/L sodium sulfate, and 0.3 mass% hydrogen peroxide). A material for a hard tissue contact device was produced by applying a voltage of -3 V (relative to the saturated calomelel electrode as a standard) to these electrodes using a potentiostat "HA-501G" (manufactured by Hokuto Denko Corporation) at 37°C for 1 hour.

### Comparative Example 1

A material for a hard tissue contact device was produced according to a similar procedure to Example except that the cathode polarization treatment was not carried out.

### Comparative Example 2

A material for a hard tissue contact device was produced according to a similar procedure to Example except that hydrogen peroxide was not contained in the electrolyte solution.

### Evaluation

In order to create a simulated living body environment, each of the materials for a hard tissue contact device produced in Example and Comparative Examples 1 and 2 was immersed in a Hanks' solution, and left to stand for one week. On day 3 after starting the immersion, the solution was replaced with a fresh Hanks' solution.

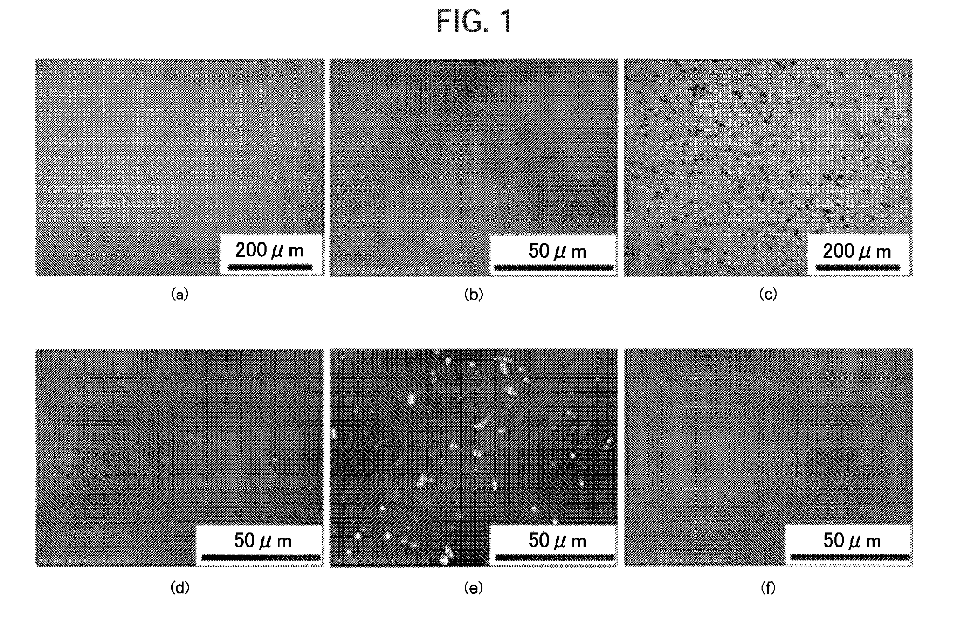

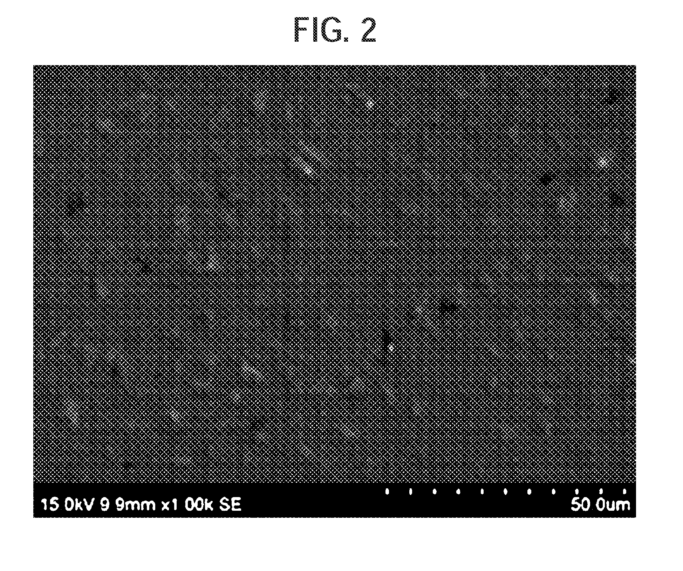

With respect to Example and Comparative Example 1, the material for a hard tissue contact device was sampled at each time point of after the mirror polishing, after the cathode polarization treatment, and after the immersion. The surface of each sample was observed with an optical microscope or a scanning electron microscope. The results are shown in Fig. 1. In addition, with respect to Comparative Example 2, the material for a hard tissue contact device after the immersion was sampled, and its surface was observed with a scanning electron microscope. The results are shown in Fig. 2.

It should be noted that in Fig. 1, (a) shows an optical micrograph of the Example after the mirror polishing; (b) shows a scanning electron micrograph of the Example after the mirror polishing; (c) shows an optical micrograph of the Example after the cathode polarization treatment; (d) shows a scanning electron micrograph of the Example after the cathode polarization treatment; (e) shows a scanning electron micrograph of the Example after the immersion; and (f) shows a scanning electron micrograph of the Comparative Example 1 after the immersion.

As shown in Fig. 1 (e), precipitation of a large amount of calcium phosphate was confirmed after the immersion on the surface of the material for a hard tissue contact device produced in the Example, whereas such precipitation of calcium phosphate was not confirmed on the surfaces of the materials for a hard tissue contact device produced in Comparative Examples 1 and 2, as shown in Fig. 1 (f) and Fig. 2.

From these results, it was revealed that precipitation of calcium phosphate on a surface treated by a cathode polarization treatment using an electrolyte solution containing hydrogen peroxide occurs in a simulated living body environment. Therefore, it was expected that biological functionality, i.e., precipitation of a large amount of calcium phosphate on the surface, can be satisfactorily imparted when implanted in a living body.

## Claims

1. A process for production of a material for a hard tissue contact device, the process comprising
subjecting an electroconductive material having a surface formed of at least one selected from the group consisting of zirconium, a zirconium compound, and a zirconium alloy to a cathode polarization treatment in an aqueous electrolyte solution containing an effective amount of hydrogen peroxide.

2. The process according to claim 1, wherein the electrolyte solution is an aqueous solution containing sulfate.

3. A hard tissue contact device formed with the material produced by the process according to claim 1 or 2.

4. A method for promoting precipitation of calcium phosphate on a surface constituted with at least one selected from the group consisting of zirconium, a zirconium compound, and a zirconium alloy in a living body environment, the method comprising: subjecting the surface to a cathode polarization treatment in an aqueous electrolyte solution containing an effective amount of hydrogen peroxide.
